# EUROPEAN PATENT SPECIFICATION

(11) **EP 1 868 254 B1**
(45) Date of publication and mention of the grant of the patent: **24.03.2010**
(21) Application number: 06115403.5
(22) Date of filing: 13.06.2006
(51) Int. Cl.: H01L 43/08

(54) **Magnetoresistance device**
Magnetowiderstandsvorrichtung
Dispositif de magnétorésistance

(43) Date of publication of application: 19.12.2007
(73) Proprietor: Hitachi Ltd., Tokyo (JP)
(72) Inventor: Williams, David, Cambridge, CB1 2EB (GB); Wunderlich, Jörg, Cambridge, CB5 8DR (GB); Troup, Andrew, Cambridge, CB5 8LJ (GB); Hasko, David, Cambridge, CB6 3SU (GB)
(74) Representative: Piotrowicz, Pawel Jan Andrzej

(56) References cited:
- EP-A2- 1 612 775
- US-A- 2006 022 672
- US-A1- 2004 129 087

## Description

The present invention relates to a magnetoresistance device particularly, but not exclusively, for use as a magnetic field sensor or a read head in a hard disk drive.

Hard disk drives (HDDs) are widely used for high-density information storage. HDDs are commonly found in computer systems traditionally associated with this type of storage, such as servers and desktop computers. However, HDDs having smaller form factors, such as 1-inch drives, can also be found in hand-held electronic devices, such as music players and digital cameras.

Higher storage capacity in HDDs can be achieved by increasing storage density. Storage density is currently doubling roughly every year and the highest storage density presently achievable using conventional technology, such as by recording data in bit cells which are arranged longitudinally in the magnetic recording medium and reading data using so-called "spin value" read heads, is about 100 Gb/in².

However, as storage density in HDDs continues to increase, then recording media and read heads encounter the problem of the superparamagnetic effect.

The superparamagnetic effect arises when the size of a ferromagnetic grain is sufficiently reduced that the energy required to change direction of magnetisation of the grain is comparable to the thermal energy. Thus, the magnetisation of the grain is liable to fluctuate and so lead to data corruption.

For recording media, a solution to the problem has been demonstrated which involves arranging bit cells perpendicularly (rather than longitudinally) to the surface of the recording medium which allows each bit cell to be large enough to avoid the superparamagnetic effect.

To address this problem in read heads, it been proposed to avoid using any ferromagnetic material and to take advantage of the so-called extraordinary magnetoresistance (EMR) effect.

A device exhibiting the EMR effect is described in "Enhanced Room-Temperature Geometric Magnetoresistance in Inhomogeneous Narrow-Gap Semiconductors", by S. A. Solin, T. Thio, D. R. Hines and J. J. Heremans, Science volume 289, p. 1530 (2000). The device is arranged in a van der Pauw configuration and includes a highly conductive gold inhomogeneity concentrically embedded in a disk of nonmagnetic indium antimonide (InSb). At zero applied magnetic field (H = 0), current flows through the gold inhomogeneity. However, at non-zero applied magnetic field (H ≠ 0), current is deflected perpendicularly to the field-line distribution, around the gold inhomogeneity and through the annulus. This gives rise to a drop in conductance.

However, this device suffers several drawbacks and so is ill suited to being used as read head. For example, the device has a configuration which is not suited to being scaled down to smaller dimensions, is heavily depleted and exhibits strong boundary and interface scattering. Furthermore, indium antimonide is an expensive material and has poor mechanical properties, which makes it difficult to process and to provide a reliable, long-lasting sensor.

Currently, high mobility narrow gap semiconductors with low carrier density, such as indium antimonide (µₙ = 7×10⁴cm²V⁻¹s⁻¹ at 300 °K), indium arsenide (µₙ = 3×10⁴cm²V⁻¹s⁻¹ at 300 °K) and gallium arsenide (µₙ = 8.5×10³cm²V⁻¹s⁻¹ at 300 °K), seem to be the best candidates for EMR-based read heads. However, these materials also tend to be expensive material and have poor mechanical properties.

Devices exhibiting the EMR effect are described in EP-A-1 612 775 and US-A-20040129087 using Indium antimonide.

The present invention seeks to provide an improved magnetoresistance device.

According to a first aspect of the present invention there is provided a magnetoresistance device having a channel comprising non-ferromagnetic semiconducting material which comprises silicon, the channel extending from a first end to a second end, a conductor which comprises a metal silicide or silicon having a higher conductivity than the channel and connecting at least two sections of the channel and a plurality of leads connected to and spaced apart along the channel.

The device can exhibit the extraordinary magnetoresistance effect even though the mobility of silicon is comparatively low (µₙ = 1.5×10³cm²V⁻¹s⁻¹ at 300 °K) compared with, for example, indium antimonide. However, using silicon has the advantage that standard silicon processing techniques can be used. Furthermore, the device is likely to be more robust, chemically, thermally and mechanically.

The channel may comprise silicon germanium. Silicon germanium can exhibit a higher mobility than silicon and so can lead to a higher magnetoresistance.

The channel may be an elongate channel. The channel may be curved. The semiconducting material may be doped with an impurity having a concentration up to 1×10¹⁷cm⁻³, up to 1×10¹⁸cm⁻³ or up to 1×10¹⁹cm⁻³. The semiconductor material may be undoped. The channel may be provided in a layer having a thickness less than 50 nm.

The conductor may be arranged laterally to the channel. The conductor may extend along the channel. The conductor may extend from the first end to the second end of the channel. The device may include an interface between the conductor and channel, the interface being broken into at least two, separate sections.

If the conductor comprises silicon, then the conductor may be doped with an impurity having a concentration of at least 1×10¹⁹ cm⁻³.

If the conductor comprises a metal silicide, then the conductor may comprise titanium silicide. Using titanium silicide can have several advantages. For example, the resistivity of titanium silicide is usually 14 - 16 µΩcm for the C54 phase at 300 °K, which compares well to the resistivities of some of the best conductors, such as gold which has a conductivity of 2.2 µΩcm. Also, if the channel is formed of silicon, then an interface between titanium silicide and silicon has a low contact resistance. Furthermore, titanium silicide can be formed by means of diffusing silicon into titanium and so can be easily formed. In addition, titanium silicide tends to exhibit good resistance to electromigration.

The conductor may be provided in a layer having a thickness less than 50 nm. The leads may extend laterally from the channel. The conductor may be connected to the channel along a first side of the channel. At least some of the leads may be connected to the channel on a second, opposite side of the channel. At least some of the leads may be connected to the channel on an upper or lower surface of the channel.
The device may comprise first, second, third and fourth leads arranged in order along channel. The first and second leads may be spaced apart by a first separation, the second and third leads may be spaced apart by a second separation less than the first separation and the third and fourth leads may be spaced apart by third separation less than the first separation.The leads may comprise semiconducting material. The leads may comprise the semiconducting material comprising the channel. The leads and the channel may be unitary. The leads may comprise silicon. The leads may comprise a metal silicide, such as titanium silicide. The leads may comprise a non-ferromagnetic metal or alloy. The leads may each have a thickness less than 50 nm.

The channel may have a width less than 100 nm and/or a length less than 10 µm. The conductor may have a width up to 500 nm and/or a length less than 10 µm. The leads may each have a width up to 200 nm, the width being in a direction which corresponds to length for the channel.

The device may further comprise a base comprising insulating material, wherein the channel, conductor and leads lie on the base. The device may further comprise a substrate, the base being disposed on the substrate. The device may further comprise contact regions arranged in the substrate between the leads and include channels between the contact regions under the leads so as to provide field effect transistors. Thus, gain-producing capability can easily be integrated into the device.

The device may further comprise a cap comprising insulating material, wherein the cap lies over the channel, region and leads. For example, a thin (e.g. 2 nm) cap of silicon dioxide can be used, which helps to protect the device while also allowing it to be placed close to a magnetic field source, such as a hard disk platen.

The device may further comprise circuitry for controlling the device. The circuitry may be configured to drive current between non-adjacent leads and to measure a voltage developed between a lead between the non-adjacent leads and another lead.

According to a second aspect of the present invention there is provided a read head for a hard disk drive comprising the magnetoresistance device.

According to a third aspect of the present invention there is provided a hard disk drive including at least one read head.

According to a fourth aspect of the present invention there is provided a method of fabricating a magnetoresistance device, the method comprising forming a channel comprising non-ferromagnetic semiconducting material comprising silicon, the channel extending between first and second ends, and connecting at least two sections of the channel using a conductor comprising a metal silicide and connecting to the channel a plurality of leads spaced apart along the channel, wherein forming the channel, connecting the at least two sections of the channel and connecting the plurality of leads to the channel comprises patterning said layer so as to define a patterned layer having a first region corresponding to the conductor, a second region corresponding to the channel and further regions corresponding to the leads, providing a metal, for example titanium, over the first region and annealing so as to form a metal silicide, for example titanium silicide, in said first region.

According to a fifth aspect of the present invention there is provided a method of fabricating a magnetoresistance device, the method comprising forming a channel comprising non-ferromagnetic semiconducting material comprising silicon, the channel extending from a first end to a second end, connecting at least two sections of the channel using a conductor comprising silicon having a conductivity higher than the channel and connecting to the channel, a plurality of leads spaced apart along the channel, wherein forming the channel, connecting the at least two sections of the channel and connecting the plurality of leads to the channel comprises providing a layer of silicon, patterning said layer so as to define a patterned layer having a first region corresponding to the conductor, a second region corresponding to the channel and further regions corresponding to the leads and doping said the first region.
According to a sixth aspect of the present invention there is provided a method of operating a magnetoresistance device having a channel which comprises silicon, the channel extending from a first end to a second end, a conductor comprising a metal silicide or silicon having a higher conductivity than the channel and connecting at least two sections of the channel and a plurality of leads connected to and spaced apart along the channel, the method comprising driving current between non-adjacent leads and measuring a voltage developed between a lead between the non-adjacent leads and another lead.

Driving the current between non-adjacent leads may comprise driving a current having a magnitude of at least 1 µA. The method may comprise applying a magnetic field to the device.

Embodiments of the present invention will now be described, by way of example, with reference to the accompanying drawings in which:
Figure 1 is a perspective view of an embodiment of a magnetoresistance device according to the present invention;
Figure 2a is a schematic view of a first circuit configuration for measuring the device shown in Figure 1;
Figure 2b illustrates a theoretical current-voltage characteristic for the circuit configuration shown in Figure 2a;
Figure 2c illustrates a measured current-voltage characteristic at 300 °K obtained using the circuit configuration shown in Figure 2a;
Figure 3a is a schematic view of a second circuit configuration for measuring the device shown in Figure 1;
Figure 3b illustrates a theoretical current-voltage characteristic for the circuit configuration shown in Figure 3a;
Figure 3c illustrates a measured current-voltage characteristic at 300 °K obtained using the circuit configuration shown in Figure 3a;
Figure 4 is a graph of resistance against applied magnetic field;
Figures 5a to 5d are perspective views of the device of Figure 1 at different stages during its fabrication;
Figures 6a to 6c are micrographs of the device of Figure 1 at different stages during its fabrication;
Figure 7a is a plan view of another embodiment of a magnetoresistance device according to the present invention;
Figure 7b is a cross sectional view of the embodiment shown in Figure 7a, taken along the line A - A';
Figure 8 is a schematic view a hard disk drive including a magnetoresistance device in accordance with the present invention; and
Figure 9 illustrates a modification to the device shown in Figure 1.

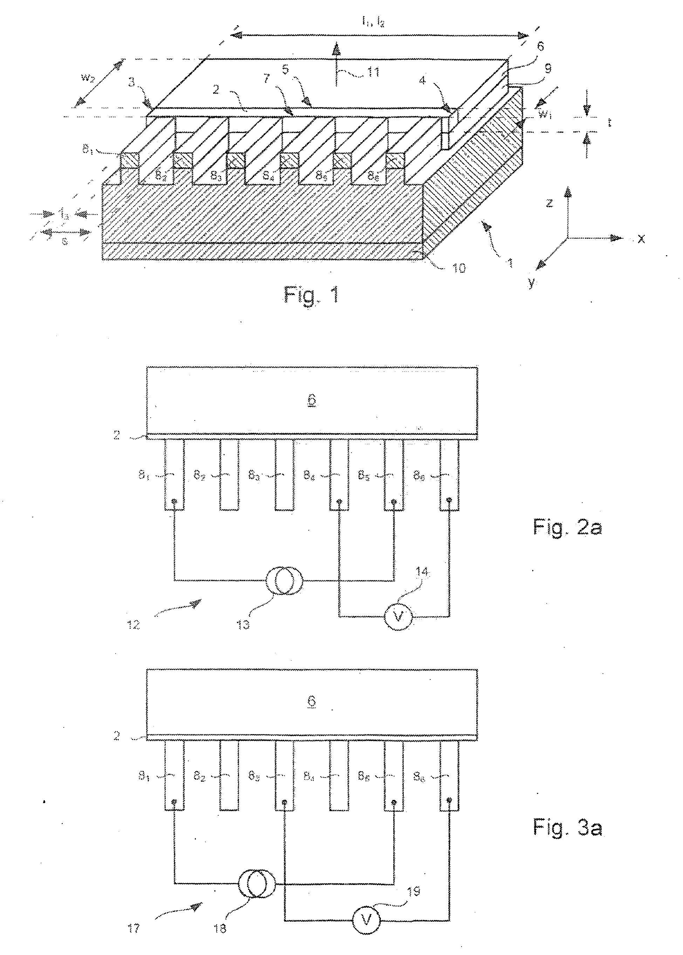

Referring to Figure 1, a first embodiment of a magnetoresistance device 1 according to the present invention is shown. The device 1 includes a channel 2 formed of n-type polycrystalline silicon (Si) doped with phosphorous (P) to a concentration of 6.4×10¹⁹cm⁻³ and having a thickness, t, of 37 nm. The channel 2 extends between first and second ends 3, 4 and has connected thereto, along a first side 5, a conductor or region 6 formed of titanium silicide (TiSi₂) having a thickness of about 40 nm and, on a second, opposite side 7, first, second, third, fourth, fifth and sixth leads 8₁, 8₂, 8₃, 8₄, 8₅, 8₆ also formed of titanium silicide and spaced apart along the channel 2. The first, second, third, fourth, fifth and sixth leads 8₁, 8₂, 8₃, 8₄, 8₅, 8₆ are arranged in order along the channel 2 with the first lead 8₁ closest to the first end 3 (in Figure 1 shown as the left-hand edge) and the sixth lead 8₆ closest to the other (i.e. second) end 4. The region 6 connects at least two portions of channel and is herein referred to as a "shunt". The channel 2, shunt 6 and leads 8₁, 8₂, 8₃, 8₄, 8₅, 8₆ are arranged on a base 9 formed of silicon dioxide (SiO₂) having an (unetched) thickness of 400 nm and is, in turn, arranged on a p-type silicon substrate 10.

In this example, the shunt 6 and the leads 8₁, 8₂, 8₃, 8₄, 8₅, 8₆ are connected to the sides 5, 7 of the channel 2, i.e. laterally. However, the shunt 6 and/or the leads 8₁, 8₂, 8₃, 8₄, 8₅, 8₆ can be connected to upper and/or lower surfaces of the channel, i.e. across the top or across the bottom of the channel 2.

The channel 2 is elongated and rectangular in plan view having a length, l₁, of about 17 µm and a width, w₁, of about 1 µm. The shunt 6 is also elongated and rectangular in plan view having a length, l₂, of about 17 µm and a width, w₂, of 4 µm. The leads 8₁, 8₂, 8₃, 8₄, 8₅, 8₆ each have a width, l₃, i.e. length along the channel 2, of 460 nm and are spaced apart having spacing, s, (i.e. pitch) of about 2.5 µm. In Figure 1, longitudinal and transverse axes of the channel 2 are shown as x- and y-axes respectively and the crystal growth axis is shown as the z-axis.

The magnetoresistance device 1 exhibits the extraordinary magnetoresistance (EMR) effect and is suitable for detecting a magnetic field 11 passing (or having a component) directed along an axis passing perpendicularly to a plane in which the channel 2 and shunt 6 are connected, e.g. along the crystal growth axis (z-axis).

Referring to Figure 2a, a first circuit configuration 12 for measuring the magnetoresistance device 1 is shown. The circuit configuration 12 includes a current source 13 configured to drive current I through the channel 2 between the first lead 8₁ and the fifth lead 8₅ and a voltmeter 14 configured to measure voltage V developed across the fourth and sixth leads 8₄, 8₆.

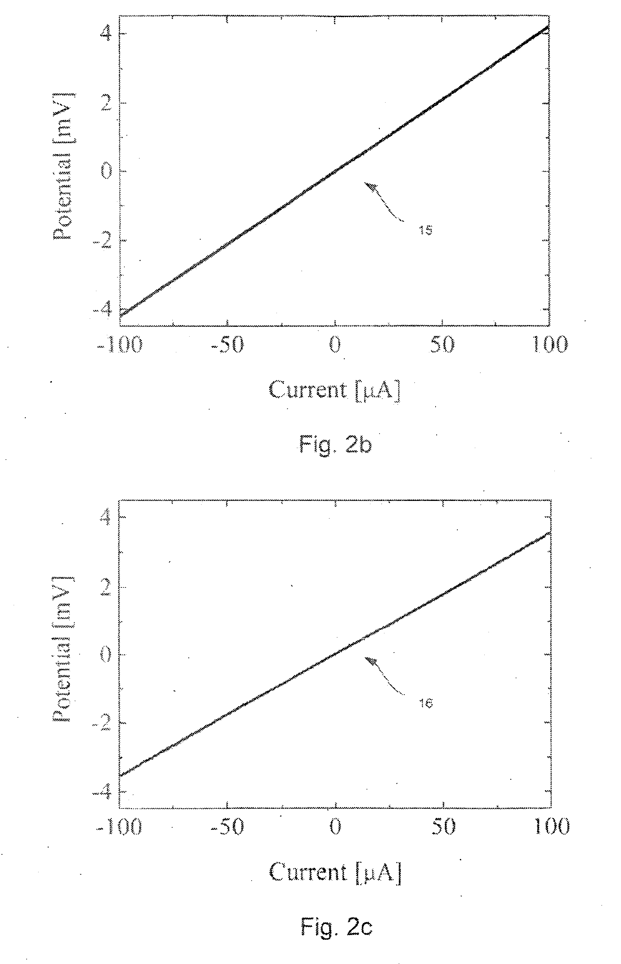

Referring to Figure 2b, a graph illustrating a first theoretical current-voltage characteristic 15 for the device 1 for the first circuit configuration 12 (Figure 2a) is shown. The device is modelled as a two-dimensional slice using finite element modelling. No fitting parameters are required in the modelling. The modelling calculates the current density and potential distribution though the device, which can be monitored using various probe configurations.

Referring to Figure 2c, a graph illustrating a first measured current-voltage characteristic 16 of the device 1 at 300 °K using the first circuit configuration 12 (Figure 2a) is shown. A measured value of resistance can be found by dividing the measured voltage, V, by the applied current, I.

Referring to Figure 3a, a second circuit configuration 17 for measuring the magnetoresistance device 1 is shown. The circuit configuration 17 includes a current source 18 configured to drive current, I, through the channel 2 between the first lead 8₁ and the fifth lead 8₅ and a voltmeter 19 configured to measure voltage, V, appearing across the third and sixth leads 8₃, 8₆.

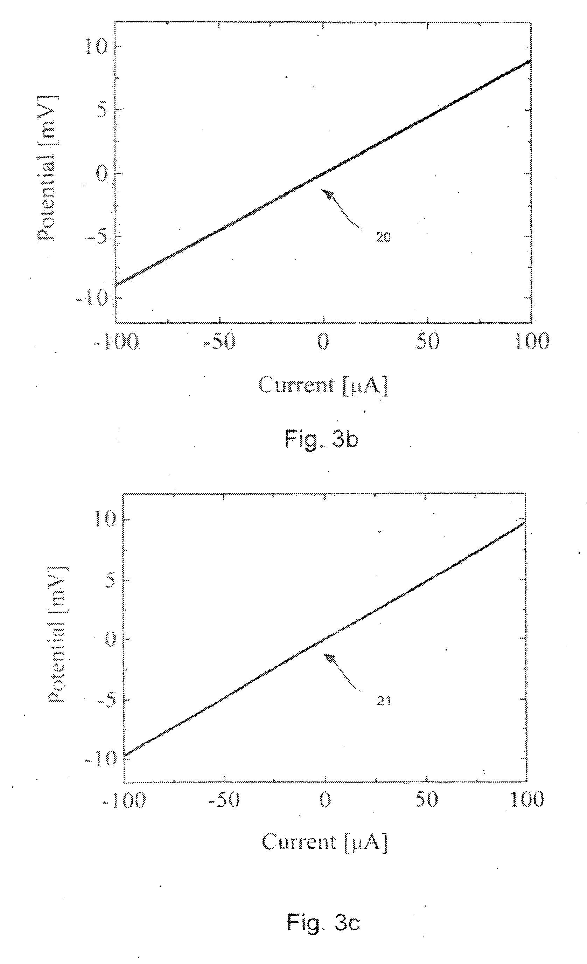

Referring to Figure 3b, a graph illustrating a second theoretical current-voltage characteristic 20 for the device 1 for the second circuit configuration 17 (Figure 3a) is shown. The device is also modelled as a two-dimensional slice using finite element modelling.

Referring to Figure 3c, a graph illustrating a second measured current-resistance characteristic 21 of the device 1 at 300 °K using the second circuit configuration 17 (Figure 3a) is shown. A measured value of resistance is found by dividing the measured voltage V by the applied current I.

Either circuit configuration 12 (Figure 2a), 17 (Figure 3a) can be used to measure a magnetic field 11 (Figure 1). The magnitude and orientation of the applied magnetic field 11 (Figure 1) affects the measured potential. In particular, if a more positive magnetic field 11 (Figure 1) is applied, then a lower potential is measured. The device 1 exhibits a change in magnetoresistance of 15.3 % between no applied field and an applied field of 10 T.

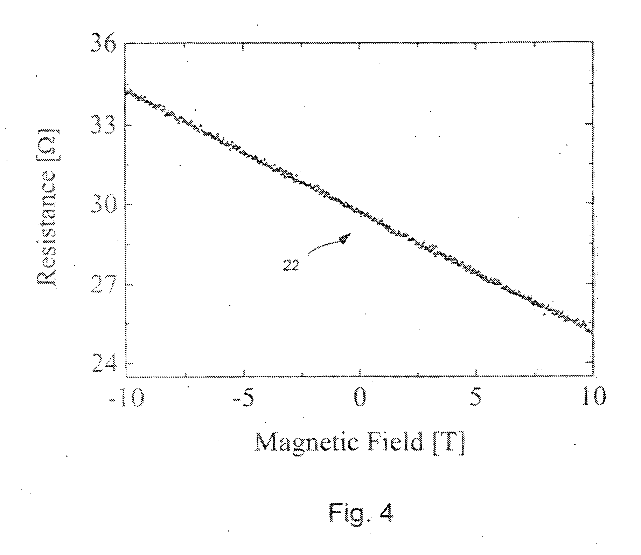

Referring to Figure 4, a graph illustrating a measured resistance-magnetic field characteristic 22 of the device 1 at 300 °K using the first circuit configuration 12 (Figure 2a) is shown.

In the first and second circuit configurations 12 (Figure 2a), 17 (Figure 3a), current is driven and voltage is measured. However, other circuit configurations may used which have a voltage source (not shown) arranged to apply a bias between two leads and a current detector (not shown) for measuring the current flowing through the channel 2 between the leads or another two leads.

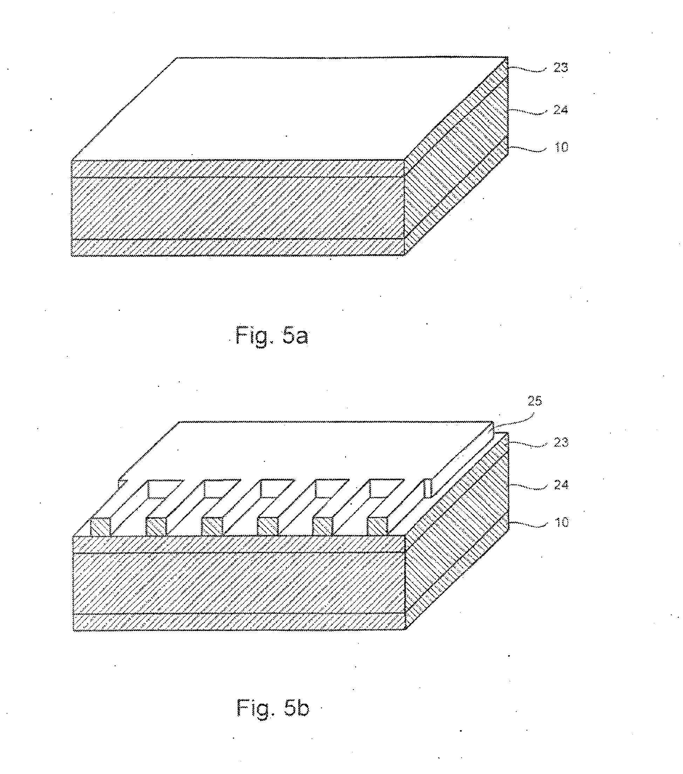

Referring to Figures 5a to 5d, a method of fabricating the device 1 will now be described.

Referring in particular to Figure 5a, a silicon-on-insulator wafer is provided having a silicon layer 23, a buried silicon dioxide layer 24 and substrate 10. The silicon layer 23 has a thickness of 37 nm and is doped with phosphorous to a concentration of 6.4×10¹⁹cm⁻³. The silicon dioxide layer 24 has a thickness of 400 nm. A naturally forming silicon dioxide layer overlying silicon layer 23 and which is usually referred to as the "surface oxide" is omitted from Figures 5a to 5d for clarity.

The silicon-on-insulator wafer is divided into chips and a chip is processed in the following way:

The chip is cleaned using a 3:1 H₂SO₄:H₂O₂ (commonly known as a "Piranha etch"), followed by a dip in 2:5:3 NH₂F:C₂H₄O₂:H₂O (also known as a "SILOX etch"). A layer of polymethylmethacrylate (PMMA) is applied (e.g. spun-on) to an upper surface of the chip and cured by baking. The PMMA layer is patterned using a scanning electron beam and developed using a mixture of IPA and water to leave a patterned PMMA layer. The chip is given a short, for example 3-minute, oxygen plasma ash, then a 30-nm thick layer of aluminium is thermally evaporated over the PMMA-patterned surface of the chip. The developed resist is "lifted-off" in acetone, then rinsed in IPA to leave an aluminium etch mask 25. The corresponding structure of the device at this stage is shown in Figure 5b.

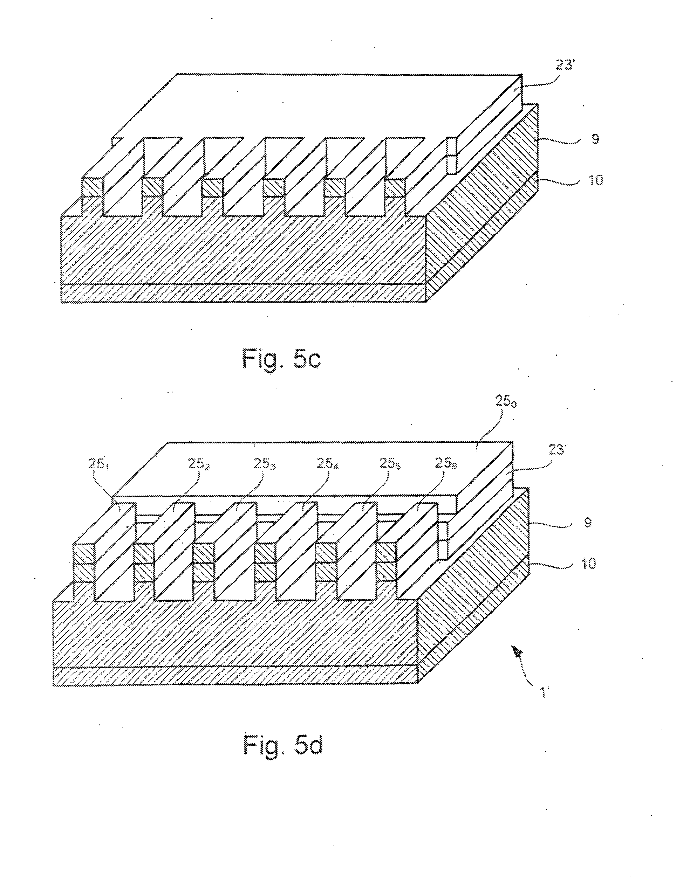

Exposed areas of the silicon and silicon dioxide layers 23, 24 are etched by reactive ion etching (RIE) using a mixture of carbon tetrafluoride and silicon tetrachloride (CF₄:SiCl₄) as a feed gas. The aluminium etch mask 25 is removed using a base, such as (CH₃)₄NOH. The corresponding structure of the device at this stage is shown in Figure 5c and shows a continuous patterned silicon layer 23' and a partially etched silicon dioxide layer 9.

The chip is cleaned using acetone and IPA. The chip is exposed to an adhesion promoter (in vapour form), in this case hexamethyldisilizane (HMDS). Another layer of PMMA is applied to the patterned surface of the chip and cured. The PMMA layer is patterned using a scanning electron beam and developed using IPA/water to leave another patterned PMMA layer. The chip is given another short oxygen plasma ash. The surface oxide (not shown) is removed using a SILOX etch and a layer of titanium having a thickness of 40 nm is sputtered over the PMMA-patterned surface of the chip. The developed resist is lifted-off, and rinsed in IPA to leave regions 25₀, 25₁, 25₂, 25₃, 25₄, 25₅, 25₆ of titanium overlying areas of the patterned silicon layer 23' which will form the shunt 6 and the leads 8₁, 8₂, 8₃, 8₄, 8₅, 8₆. The corresponding structure of the device at this stage is shown in Figure 5d.

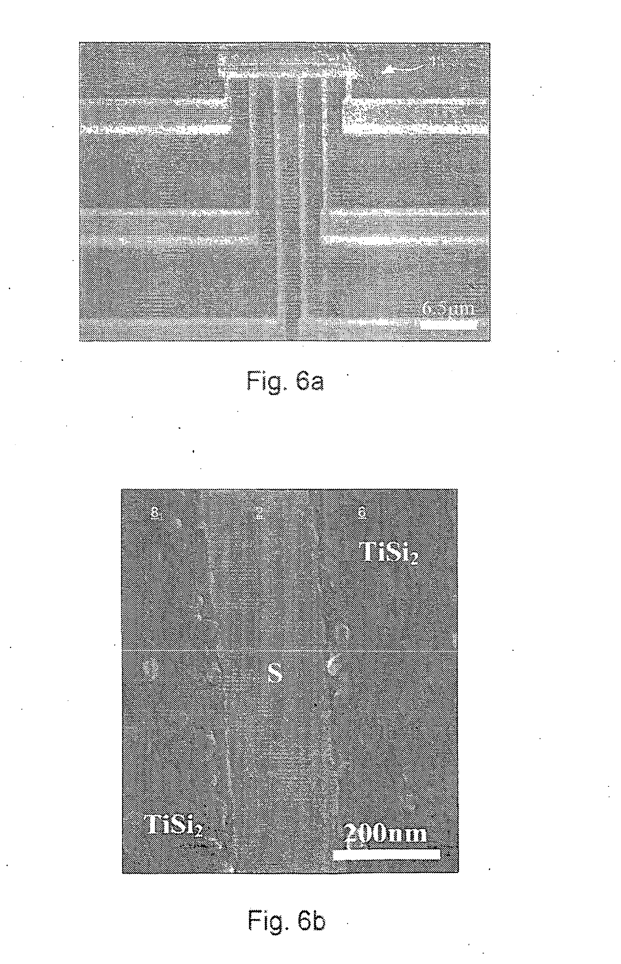

The chip is annealed at 700 °C in an inert atmosphere, such as dry nitrogen. Unreacted titanium is removed using a Piranha etch. The corresponding structure of the device at this stage is shown in Figure 1. Figure 6a is an optical micrograph of the device at this stage. Figure 6b is an electron micrograph of part of the channel 2.

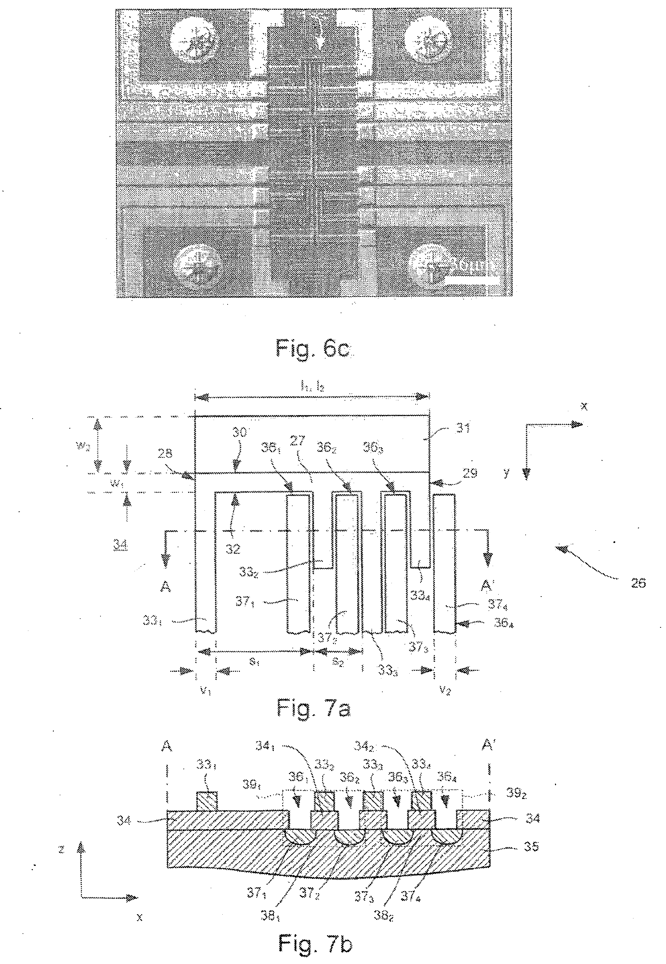

The chip is cleaned using acetone and IPA. A layer of optical resist is spun-on. The optical resist layer is patterned using a mask (which is also referred to as a reticle) and a UV light source and developed using an optical resist developer to leave a patterned optical resist layer. The chip is given a short, for example 30 s, oxygen plasma ash, then successive layers of chromium and gold are thermally evaporated over the resist-patterned surface of the chip. The resist is lifted-off in acetone, then rinsed in IPA to leave gold bond pads (not shown). Figure 6c is an optical micrograph of the device 1.

Referring to Figures 7a and 7b, a second embodiment of a magnetoresistance device 26 according to the present invention is shown. The device 26 includes a channel 27 formed of n-type polycrystalline Si doped with P to a concentration of 1 × 10¹⁷cm⁻³ and having a thickness of about 40 nm. The channel 27 extends between first and second ends 28, 29 and has connected thereto, along a first side 30, a shunt 31 formed of n-type polycrystalline Si, doped with P to a concentration of 1×10²¹cm⁻³ and having a thickness of about 40 nm and, on a second, opposite side 32, first, second, third and fourth leads 33₁, 33₂, 33₃, 33₄ also formed of n-type polycrystalline Si doped with P to a concentration of 1 × 10²¹cm⁻³ and spaced apart along the channel 27. The first, second, third, fourth leads 33₁, 33₂, 33₃, 33₄ are arranged in order along the channel 27.

Different doping concentrations in the channel 27, shunt 31 and leads 33₁, 33₂, 33₃, 33₄ can be achieved by depositing a diffusion barrier layer (not shown), e.g. silicon nitride, over a patterned layer of undoped silicon, opening windows (not shown) in the diffusion barrier layer over regions corresponding to the shunt and leads, then ion-implanting or gaseously diffusing dopant, such as phosphorous, through the windows and into the regions corresponding to the shunt and leads. The diffusion barrier layer is removed by wet etching and the sample annealed.

Alternatively, different doping concentrations in the channel 27, shunt 31 and leads 33₁, 33₂, 33₃, 33₄ can be achieved by scanning ion beam implantation and annealing.

The channel 27, shunt 31 and leads 33₁, 33₂, 33₃, 33₄ are arranged on an insulating base 34 formed of SiO₂ having a thickness of 10 nm and is, in turn, arranged on a p-type silicon substrate 35. The base 34 includes windows 36₁, 36₂, 36₃, 36₄ which are used during fabrication to define first, second, third and fourth regions 37₁, 37₂, 37₃, 37₄ of n-type silicon (often referred to as "diffusion wells" or simply "wells") in the p-type silicon substrate 35.

The channel 27 has a length, l₁, of about 1 µm and a width, w₁, of about 50 nm. The shunt 31 has a length, l₂, of about 1 µm and a width, w₂, of about 0.2 µm. The leads 33₁, 33₂, 33₃, 33₄ each have a width, v₁, of 200 nm. The first and second leads 33₁, 33₂ are spaced apart by a first separation (i.e. pitch), s₁, of 0.75 µm. The windows 36₁, 36₂, 36₃, 36₄ each have a width, v₂, of about 200 nm. The others leads 33₂, 33₃, 33₄ are spaced apart from neighbouring leads by a second separation, s₂, of 0.1 µm. In some embodiments of the invention, the second separation s₂ is made as small as possible and may correspond to the minimum feature size, F.

The first and second n-type wells 37₁, 37₂ provide source and drain regions for a first channel 38₁ running between the wells 37₁, 37₂. The second lead 33₂ overlies and is separated from the first channel 38₁ by a first portion 34₁ of the insulating base 34, thereby providing a gate electrode and a gate oxide respectively for a first metal-oxide-semiconductor field-effect transistor (MOSFET) 39₁. Likewise, the third and fourth n-type wells 37₃, 37₄, a second channel 38₂, a second gate oxide 34₂ and the fourth lead 33₄ provide a second MOSFET 39₂.

During operation, a current, I, is driven through the channel 27 between the first and third leads 33₁, 33₃. Biases are applied between source and drain regions 37₁, 37₂, 37₃, 37₄ of each MOSFETs 39₁, 39₂ and the respective source-drain currents, I_{SD}, measured so as to determine potentials of the second and fourth leads 33₂, 33₄.

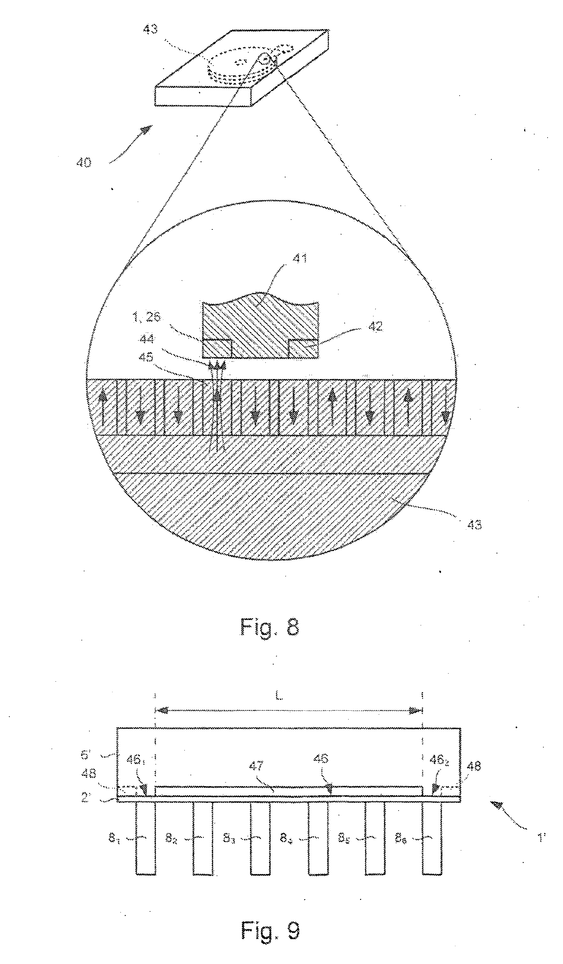

Referring to Figure 8, the device 1, 26 is useable as a read head in a hard disk drive 40. A slider 41 supports the device 1, 26 (inverted with respect to the configuration shown in Figures 1 a or 7b) and a write head 42 over a rotatable platen 43. The device 1, 26 measures magnetic field 44 produced by a perpendicularly-arranged bit cell 45 passing beneath it. The device 1, 26 may be used in a hard disk drive having longitudinally-arranged bit cells.

Referring to Figure 9, a third embodiment of a magnetoresistance device 1' according to the present invention is shown. The device 1' is a modification of the device 1 shown in Figure 1.

An interface 46 between the shunt 6' and channel 2' may be broken into at least two, separate sections 46₁, 46₂ by at least one region 47 without conducting material or of non-conductive material. For example, the (or each) region 47 may be a void cut using ion milling or a region of damaged material formed by ion implantation. This can enhance the magnetoresistance.

In this example, the region 47 has a length L such that the shunt 6' contacts the channel 2' at least at the same point (i.e. length) along channel 2' at which the outermost leads 8₁, 8₆ are connected to the channel 2'. Additional regions 48 of non-conductive material may be provided to limit further the length of contact along the channel 2' so that the shunt 6' contacts the channel 2' only at the same points along channel 2' at which the outermost leads 8₁, 8₆ are connected to the channel 2'. Alternatively, the shunt 6' may contact two points proximate to distal ends of the channel 2'.

The device 26 (Figures 7a and 7b) can be modified in a similar way.

It will be appreciated that many modifications may be made to the embodiments hereinbefore described. The channel need not be straight, but may be curved in-plane. The device may be a silicon-based device, for example the channel, shunt and/or the leads may comprise a silicon-containing material, such as silicon or silicon-germanium (e.g. Si_{0.9}Ge_{0.1}). Different silicon-containing materials can be used in different parts of the device. The channel may comprise silicon germanium. The channel may be undoped or doped with an impurity (n-type or p-type) up to a concentration of 1×10¹⁷cm⁻³, up to a concentration of 1×10¹⁸cm⁻³, up to a concentration of 1×10¹⁹cm⁻³ or up to a concentration of 1×10²⁰cm⁻³. The channel may be provided in a layer having a different thickness to those described earlier, for example having a thickness less than 50 nm, preferably between 5 and 40 nm. The shunt can be arranged above and/or below the channel, i.e. underlie and/or overlie the channel, and contact top and/or bottom sides of the channel. Rearranging the configuration of shunt relative to the channel may change the axis along which the device senses a magnetic field. The shunt may extend along a portion of the channel, i.e. less than the full length of the channel. The shunt may be polygonal in plan view. The shunt may include more than one discrete part, e.g. in the form a broken line of conducting regions along the channel. The shunt may comprise semiconducting material having a higher conductivity than the semiconducting material comprising the channel. The semiconducting material comprising the shunt may be doped with an impurity having a concentration of at least 1×10¹⁹cm⁻³, for example 1×10²¹cm⁻³, and may comprise one or more δ-doped layers. The shunt may comprise a metal silicide and need not be titanium silicide. An n-type impurity, such as phosphorous, (or, in some cases, a p-type impurity) may be implanted or diffused into the surface of the patterned silicon layer before depositing the metal for forming a metal silicide shunt so as to improve contact between the semiconductor and the silicide. The shunt may comprise a non-ferromagnetic material, such as a non-ferromagnetic metal or alloy, such as aluminium. The shunt may be provided in a layer having a different thickness, for example less than 50 nm. The leads may comprise a semiconducting material and may be doped with an impurity (n-type or p-type) up to a concentration of different, e.g. higher, than the semiconducting material in the channel. The leads need not be formed of titanium silicide, but may comprise a metal silicide. The leads may comprise a non-ferromagnetic metal or alloy. The leads may each have a thickness less than 50 nm. The channel may have a width (i.e. w₁) less than 100 nm and/or a length (i.e. l₁) less than 10 µm. The shunt may have a width (i.e. w₂) up to 500 nm and/or a length (i.e. l₂) less than 10 µm which may or may not be the same as the length of the channel. The leads may each have a width (i.e. l₃) up to 200 nm, the width being in a direction which corresponds to length for the channel. The leads need not be arranged perpendicularly with respect to the channel. End leads, for example first and sixth leads 8₁, 8₆ (Figure 1), may be arranged to approach the channel, e.g. channel 2 (Figure 1), from the ends, e.g. ends 3, 4 (Figure 1) of the channel, rather than transversely. At least some of the leads can be arranged above and/or below the channel, i.e. underlie and/or overlie the channel. The shunt and the leads need not be arranged on opposite sides (or surfaces) of the channel. Other concentrations and mixtures for etches and developers may be used. Other etches, resists and developers may be used. Etching, exposure and development time can be varied and can be found by routine experiment. The anneal temperature may also be found by routine experiment.

## Claims

1. A magneto resistance device (1; 26) having a channel comprising non-ferromagnetic semiconducting material (2; 27) which comprises silicon, the channel extending from a first end (3; 28) to a second end (4; 29), a conductor (6; 31) which comprises a metal silicide or silicon having a higher conductivity than the channel and connecting at least two sections of the channel and a plurality of leads (8₁, 8₂, 8₃, 8₄, 8₅, 8₆; 33₁, 33₂, 33₃, 33₄) connected to and spaced part along the channel.

2. A device according to claim 1, wherein the channel (2; 27) is an elongate channel.

3. A device according to claim 1 or 2, wherein the channel (2; 27) is curved.

4. A device according to any preceding claim, wherein the channel (2; 27) comprises silicon germanium.

5. A device according to any preceding claim, wherein the semiconducting material of the channel is doped with an impurity having a concentration up to 1×10¹⁷ cm⁻³.

6. A device according to any preceding claim, wherein the semiconducting material of the channel is doped with an impurity having a concentration up to 1×10¹⁸cm⁻³.

7. A device according to any preceding claim, wherein the semiconducting material of the channel is doped with an impurity having a concentration up to 1×10¹⁹cm⁻³.

8. A device according to any one of claims 1 to 4, wherein the semiconductor material is undoped.

9. A device according to any preceding claim, wherein the channel (2; 27) is provided in a layer having a thickness less than 50 nm.

10. A device according to any preceding claim, wherein the conductor (6; 31) is arranged laterally to the channel (2; 27).

11. A device according to any preceding claim, wherein the conductor (6; 31) extends along the channel (2; 27).

12. A device according to any preceding claim, wherein the conductor (6; 31) extends from the first end (3; 28) to the second end (4; 29) of the channel (2; 27).

13. A device according to any preceding claim, comprising an interface between the conductor (6; 31) and channel (2; 27), the interface being broken into at least two, separate sections.

14. A device according to any preceding claim, wherein the conductor (6; 31) comprises semiconducting material.

15. A device according to any preceding claim, wherein, if the conductor comprises silicon, the conductor (6; 31) is doped with an impurity having a concentration of at least 1×10¹⁹cm⁻³.

16. A device according to any preceding claim, wherein, if the conductor comprises a metal silicide, the conductor (6; 31) comprises titanium silicide.

17. A device according to any preceding claim, wherein the conductor (6; 31) is provided in a layer having a thickness less than 50 nm.

18. A device according to any preceding claim, wherein the leads (8₁, 8₂, 8₃, 8₄, 8₅, 8₆; 33₁, 33₂, 33₃, 33₄) extend laterally from the channel.

19. A device according to any preceding claim, wherein the conductor (6; 31) is connected to the channel (2, 27) along a first side (5; 30) of the channel.

20. A device according to claim 19, wherein at least some of the leads (8₁, 8₂, 8₃, 8₄, 8₅, 8₆; 33₁, 33₂, 33₃, 33₄) are connected to the channel (2; 37) on a second, opposite side (7; 32) of the channel.

21. A device according to claim 19 or 20, wherein at least some of the leads (8₁, 8₂, 8₃, 8₄, 8₅, 8₆; 33₁, 33₂, 33₃, 33₄) are connected to the channel (2; 37) on an upper or lower surface of the channel.

22. A device according to any preceding claim, comprising first, second, third and fourth leads (8₁, 8₂, 8₃, 8₄, 8₅, 8₆; 33₁, 33₂, 33₃, 33₄) arranged in order along channel.

23. A device according to claim 22, wherein the first and second leads (33₁, 33₂) are spaced apart by a first separation (s₁), the second and third leads (33₂, 33₃) are spaced apart by a second separation (s₂) less than the first separation and the third and fourth leads (33₃, 33₄) are spaced apart by third separation less than the first separation.

24. A device according to any preceding claim, wherein the leads (33₁, 33₂, 33₃, 33₄) comprise semiconducting material.

25. A device according to claim 23, wherein the leads (33₁, 33₂, 33₃, 33₄) comprise the semiconducting material comprising the channel (27).

26. A device according to claim 24, wherein the leads (33₁, 33₂, 33₃, 33₄) and the channel (27) are unitary.

27. A device according to any one of claims 24 to 26, wherein the leads (33₁, 33₂, 33₃, 33₄) comprise silicon.

28. A device according to any one of claims 1 to 23, wherein the leads (8₁, 8₂, 8₃, 8₄, 8₅, 8₆) comprise metal silicide.

29. A device according to any one of claims 1 to 23, wherein the leads (8₁, 8₂, 8₃, 8₄, 8₅, 8₆) comprise titanium silicide.

30. A device according to anyone of claims 1 to 23, wherein the leads comprise a non-ferromagnetic metal or alloy.

31. A device according to any preceding claim, wherein the leads each have a thickness less than 50 nm.

32. A device according to any preceding claim, wherein the channel (2; 27) has a width less than 100 nm.

33. A device according to any preceding claim, wherein the channel (2; 27) has a length less than 10 µm.

34. A device according to any preceding claim, wherein the conductor (6; 31) has a width up to 500 nm.

35. A device according to any preceding claim, wherein the conductor (6; 31) has a length less than 10 µm.

36. A device according to any preceding claim, wherein the leads (8₁, 8₂, 8₃, 8₄, 8₅, 8₆; 33₁, 33₂, 33₃, 33₄) each have a width up to 200 nm, the width being in a direction which corresponds to length for the channel.

37. A device according to any preceding claim, further comprising a base (9; 34) comprising insulating material, wherein the channel (2; 27), conductor (6; 31) and leads (8₁, 8₂, 8₃, 8₄, 8₅, 8₆; 33₁, 33₂, 33₃, 33₄) lie on the base.

38. A device according to claim 37, further comprising a substrate (10; 35), the base (9; 34) disposed on the substrate.

39. A device according to claim 38, further comprising contact regions (37₁, 37₂, 37₃, 37₄) arranged in the substrate (35) between the leads (33₁, 33₂, 33₃, 33₄) and channels (38₁, 38₂) between the contact regions under the leads so as to provide field-effect transistors (39₁, 39₂).

40. A device according to any preceding claim, further comprising a cap comprising insulating material, wherein the cap lies over the channel, region and leads.

41. A device according to any preceding claim, further comprising circuitry (12) for controlling the device.

42. A device according to claim 41, wherein the circuitry (12) is configured to drive current between non-adjacent leads (8₁, 8₅) and to measure a voltage developed between a lead (8₃) between the non-adjacent leads and another lead (8₆).

43. A read head (1; 26) for a hard disk drive (40) comprising the magnetoresistance device according to any preceding claim.

44. A hard disk drive (40) including at least one read head (1; 26) according to claim 43.

45. A method of fabricating a magnetoresistance device, the method comprising:
forming a channel comprising non-ferromagnetic semiconducting material (2; 27) which comprises silicon, the channel extending from a first end (3; 28) to a second end (4; 29);
connecting at least two sections of the channel using a conductor (6; 31) comprising non-ferromagnetic material; and
connecting to the channel, a plurality of leads (8₁, 8₂, 8₃, 8₄, 8₅, 8₆; 33₁, 33₂, 33₃, 33₄) spaced apart along the channel,
wherein forming the channel, connecting the at least two sections of the channel and connecting the plurality of leads to the channel comprises:
providing a layer (23) of silicon;
patterning said layer so as to define a patterned layer (23') having a first region corresponding to the conductor (6; 31), a second region corresponding to the channel (2; 27) and further regions corresponding to the leads (8₁,8₂, 8₃, 8₄, 8₅, 8₆; 33₁, 33₂, 33₃, 33₄);
providing a metal (25₀) over the first region; and
annealing so as to form a metal silicide in said first region.

46. A method of fabricating a magnetoresistance device, the method comprising:
forming a channel comprising non-ferromagnetic semiconducting material (2; 27) which comprises silicon, the channel extending from a first end (3; 28) to a second end (4; 29);
connecting at least two sections of the channel using a conductor (6; 31) comprising non-ferromagnetic material; and
connecting to the channel, a plurality of leads (8₁, 8₂, 8₃, 8₄, 8₅, 8₆; 33₁, 33₂, 33₃, 33₄) spaced apart along the channel,
wherein forming the channel, connecting the at least two sections of the channel and connecting the plurality of leads to the channel comprises:
providing a layer (23) of silicon;
patterning said layer so as to define a patterned layer (23') having a first region corresponding to the conductor (6; 31), a second region corresponding to the channel (2; 27) and further regions corresponding to the leads (8₁, 8₂, 8₃, 8₄, 8₅, 8₆; 33₁, 33₂, 33₃, 33₄); and
doping said the first region.

47. A method of operating a magnetoresistance device having a channel comprising non-ferromagnetic semiconducting material (2; 27) which comprises silicon, the channel extending from a first end (3; 28) to a second end (4; 29), a conductor (6; 31) which comprises a metal silicide or silicon having a higher conductivity than the semiconducting material and connecting at least two regions of the channel and a plurality of leads (8₁, 8₂, 8₃, 8₄, 8₅, 8₆; 33₁, 33₂, 33₃, 33₄) spaced apart along the channel, the method comprising:
driving current between non-adjacent leads (8₁, 8₅; 33_{1,} 33₃); and
measuring a voltage developed between a lead (8₃; 33₂) which is between the non-adjacent leads and another lead (8₆; 33₄).

48. A method according to claim 47, wherein driving the current between non-adjacent leads (8₁, 8₅; 33_{1,} 33₃) comprises driving a current having a magnitude of at least 1 µA.

49. A method according to claim 47 or 48, further comprising:
applying a magnetic field (11) to the magnetoresistance device.

## Patentansprüche

1. Magnetowiderstandsvorrichtung (1; 26) mit einem Kanal (2; 27), umfassend nicht-ferromagnetisches Halbleitermaterial, welches Silizium umfasst, wobei sich der Kanal von einem ersten Ende (3; 28) zu einem zweiten Ende (4; 29) erstreckt, einem Leiter (6; 31), der ein Metallsilizid oder Silizium mit einer höheren Leitfähigkeit als der Kanal umfasst und wenigstens zwei Abschnitte des Kanals verbindet, sowie einer Mehrzahl von Leitungen (8₁, 8₂, 8₃, 8₄, 8₅, 8₆; 33₁, 33₂, 33₃, 33₄), die entlang des Kanals voneinander beabstandet und mit ihm verbunden sind.

2. Vorrichtung nach Anspruch1, wobei der Kanal (2; 27) ein länglicher Kanal ist.

3. Vorrichtung nach Anspruch 1 oder 2, wobei der Kanal (2; 27) gekrümmt ist.

4. Vorrichtung nach einem der vorhergehenden Ansprüche, wobei der Kanal (2; 27) Silizium-Germanium umfasst.

5. Vorrichtung nach einem der vorhergehenden Ansprüche, wobei das Halbleitermaterial des Kanals mit einer Verunreinigung dotiert ist, die eine Konzentration von bis zu 1x10¹⁷ cm⁻³ hat.

6. Vorrichtung nach einem der vorhergehenden Ansprüche, wobei das Halbleitermaterial des Kanals mit einer Verunreinigung dotiert ist, die eine Konzentration von bis zu 1x10¹⁸ cm⁻³.

7. Vorrichtung nach einem der vorhergehenden Ansprüche, wobei das Halbleitermaterial des Kanals mit einer Verunreinigung dotiert ist, die eine Konzentration von bis zu 1x10¹⁹ cm⁻³.

8. Vorrichtung nach einem der Ansprüche 1 bis 4, wobei das Halbleitermaterial undotiert ist.

9. Vorrichtung nach einem der vorhergehenden Ansprüche, wobei der Kanal (2; 27) in einer Schicht vorgesehen ist, die eine Dicke von weniger als 50 nm aufweist.

10. Vorrichtung nach einem der vorhergehenden Ansprüche, wobei der Leiter (6; 31) seitlich vom Kanal (2; 27) angeordnet ist.

11. Vorrichtung nach einem der vorhergehenden Ansprüche, wobei sich der Leiter (6; 31) entlang des Kanals (2; 27) erstreckt.

12. Vorrichtung nach einem der vorhergehenden Ansprüche, wobei sich der Leiter (6; 31) vom ersten Ende (3; 28) zum zweiten Ende (4; 29) des Kanals (2; 27) erstreckt.

13. Vorrichtung nach einem der vorhergehenden Ansprüche, umfassend eine Grenzfläche zwischen dem Leiter (6; 31) und dem Kanal (2; 27), wobei die Grenzfläche in wenigstens zwei separate Abschnitte unterteilt ist.

14. Vorrichtung nach einem der vorhergehenden Ansprüche, wobei der Leiter (6; 31) Halbleitermaterial umfasst.

15. Vorrichtung nach einem der vorhergehenden Ansprüche, wobei dann, wenn der Leiter Silizium umfasst, der Leiter (6; 31) mit einer Verunreinigung dotiert ist, die eine Konzentration von wenigstens 1x10¹⁹ cm⁻³ aufweist.

16. Vorrichtung nach einem der vorhergehenden Ansprüche, wobei dann, wenn der Leiter ein Metallsilizid umfasst, der Leiter (6; 31) Titansilizid umfasst.

17. Vorrichtung nach einem der vorhergehenden Ansprüche, wobei der Leiter (6; 31) in einer Schicht vorgesehen ist, die eine Dicke von weniger als 50 nm aufweist.

18. Vorrichtung nach einem der vorhergehenden Ansprüche, wobei sich die Leitungen (8₁, 8₂, 8₃, 8₄, 8₅, 8₆; 33₁, 33₂, 33₃, 33₄) seitlich vom Kanal erstrecken.

19. Vorrichtung nach einem der vorhergehenden Ansprüche, wobei der Leiter (6; 31) mit dem Kanal (2; 27) entlang einer ersten Seite (5; 30) des Kanals verbunden ist.

20. Vorrichtung nach Anspruch 19, wobei wenigstens einige der Leitungen (8₁, 8₂, 8₃, 8₄, 8₅, 8₆; 33₁, 33₂, 33₃, 33₄) mit dem Kanal (2; 37) an einer zweiten, gegenüberliegenden Seite (7; 32) des Kanals verbunden sind.

21. Vorrichtung nach Anspruch 19 oder 20, wobei wenigstens einige der Leitungen (8₁, 8₂, 8₃, 8₄, 8₅, 8₆; 33₁, 33₂, 33₃, 33₄) mit dem Kanal (2; 37) an einer oberen oder einer unteren Oberfläche des Kanals verbunden sind.

22. Vorrichtung nach einem der vorhergehenden Ansprüche, umfassend erste, zweite, dritte und vierte Leitungen (8₁, 8₂, 8₃, 8₄, 8₅, 8₆; 33₁, 33₂, 33₃, 33₄), die in Reihenfolge entlang des Kanals angeordnet sind.

23. Vorrichtung nach Anspruch 22, wobei die ersten und zweiten Leitungen (33₁, 33₂) um einen ersten Abstand (s₁) voneinander beabstandet sind, die zweiten und die dritten Leitungen (33₂, 33₃) um einen zweiten Abstand (s₂) voneinander beabstandet sind, der kleiner als der erste Abstand ist, und die dritten und die vierten Leitungen (33₃, 33₄) um einen dritten Abstand voneinander beabstandet sind, der kleiner als der erste Abstand ist.

24. Vorrichtung nach einem der vorhergehenden Ansprüche, wobei die Leitungen (33₁, 33₂, 33₃, 33₄) Halbleitermaterial umfassen.

25. Vorrichtung nach Anspruch 23, wobei die Leitungen (33₁, 33₂, 33₃, 33₄) das Halbleitermaterial umfassen, das den Kanal (27) umfasst.

26. Vorrichtung nach Anspruch 24, wobei die Leitungen (33₁, 33₂, 33₃, 33₄) und der Kanal (27) einheitlich sind.

27. Vorrichtung nach einem der Ansprüche 24 bis 26, wobei die Leitungen (33₁, 33₂, 33₃, 33₄) Silizium umfassen.

28. Vorrichtung nach einem der Ansprüche 1 bis 23, wobei die Leitungen (8₁, 8₂, 8₃, 8₄, 8₅, 8₆) Metallsilizid umfassen.

29. Vorrichtung nach einem der Ansprüche 1 bis 23, wobei die Leitungen (8₁, 8₂, 8₃, 8₄, 8₅, 8₆) Titansilizid umfassen.

30. Vorrichtung nach einem der Ansprüche 1 bis 23, wobei die Leitungen ein(e) nicht-ferromagnetische(s) Metall oder Legierung umfassen.

31. Vorrichtung nach einem der vorhergehenden Ansprüche, wobei die Leitungen jeweils eine Dicke von weniger als 50 nm aufweisen.

32. Vorrichtung nach einem der vorhergehenden Ansprüche, wobei der Kanal (2; 27) eine Breite von weniger als 100 nm aufweist.

33. Vorrichtung nach einem der vorhergehenden Ansprüche, wobei der Kanal (2; 27) eine Länge von weniger als 10 µm aufweist.

34. Vorrichtung nach einem der vorhergehenden Ansprüche, wobei der Leiter (6; 31) eine Breite von bis zu 500 nm aufweist.

35. Vorrichtung nach einem der vorhergehenden Ansprüche, wobei der Leiter (6; 31) eine Länge von weniger als 10 µm aufweist.

36. Vorrichtung nach einem der vorhergehenden Ansprüche, wobei die Leitungen (8₁, 8₂, 8₃, 8₄, 8₅, 8₆; 33₁, 33₂, 33₃, 33₄) jeweils eine Breite von bis zu 200 nm aufweisen, wobei die Breite in einer Richtung liegt, die der Länge für den Kanal entspricht.

37. Vorrichtung nach einem der vorhergehenden Ansprüche, ferner umfassend eine Basis (9; 34) umfassend Isoliermaterial, wobei der Kanal (2; 27), der Leiter (6; 31) und die Leitungen (8₁, 8₂, 8₃, 8₄, 8₅, 8₆; 33₁, 33₂, 33₃, 33₄) auf der Basis liegen.

38. Vorrichtung nach Anspruch 37, ferner umfassend ein Substrat (10; 35), wobei die Basis (9; 34) auf dem Substrat angeordnet ist.

39. Vorrichtung nach Anspruch 38, ferner umfassend Kontaktbereiche (37₁, 37₂, 37₃, 37₄), die in dem Substrat (35) zwischen den Leitungen (33₁, 33₂, 33₃, 33₄) und Kanälen (38₁, 38₂) zwischen den Kontaktbereichen unter den Leitungen angeordnet sind, um Feldeffekttransistoren (39₁, 39₂) bereitzustellen.

40. Vorrichtung nach einem der vorhergehenden Ansprüche, ferner umfassend einen Deckel, umfassend Isoliermaterial, wobei der Deckel über dem Kanal, dem Bereich und den Leitungen liegt.

41. Vorrichtung nach einem der vorhergehenden Ansprüche, ferner umfassend eine Schaltung (12) zum Steuern der Vorrichtung.

42. Vorrichtung nach Anspruch 41, wobei die Schaltung (12) dazu ausgelegt ist, Strom zwischen nicht-benachbarten Leitungen (8₁, 8₅) fließen zu lassen und eine Spannung zu messen, die sich zwischen einer Leitung (8₃) zwischen den nicht-benachbarten Leitungen und einer anderen Leitung (8₆) entwickelt.

43. Lesekopf (1; 26) für einen Festplattenantrieb (40), umfassend die Magnetowiderstandsvorrichtung nach einem der vorhergehenden Ansprüche.

44. Festplattenantrieb (40), umfassend wenigstens einen Lesekopf (1; 26) nach Anspruch 43.

45. Verfahren zur Herstellung einer Magnetowiderstandsvorrichtung, wobei das Verfahren umfasst.
Bilden eines Kanals (2; 27), umfassend nicht-ferromagnetisches Halbleitermaterial, das Silizium umfasst, wobei sich der Kanal von einem ersten Ende (3; 28) zu einem zweiten Ende (4; 29) erstreckt;
Verbinden von wenigstens zwei Abschnitten des Kanals unter Verwendung eines Leiters (6; 31), der nicht-ferromagnetisches Material umfasst;
Verbinden einer Mehrzahl von Leitungen (8₁, 8₂, 8₃, 8₄, 8₅, 8₆; 33₁, 33₂, 33₃, 33₄), die entlang des Kanals voneinander beabstandet sind, mit dem Kanal,
wobei das Bilden des Kanals, das Verbinden der wenigstens zwei Abschnitte des Kanals und das Verbinden der Mehrzahl von Leitungen mit dem Kanal umfassen:
Bereitstellen einer Schicht (23) aus Silizium;
Bemustern der Schicht derart, dass eine gemusterte Schicht (23') definiert wird, mit einem ersten Bereich, der dem Leiter (6; 31) entspricht, einem zweiten Bereich, der dem Kanal (2; 27) entspricht und weiteren Bereichen, die den Leitungen (8₁, 8₂, 8₃, 8₄, 8₅, 8₆; 33₁, 33₂, 33₃, 33₄) entsprechen;
Bereitstellen eines Metalls (25₀) über dem ersten Bereich;
Tempern, um ein Metallsilizid in dem ersten Bereich zu bilden.

46. Verfahren zur Herstellung einer Magnetowiderstandsvorrichtung, wobei das Verfahren umfasst.
Bilden eines Kanals (2; 27), umfassend nicht-ferromagnetisches Halbleitermaterial, das Silizium umfasst, wobei sich der Kanal von einem ersten Ende (3; 28) zu einem zweiten Ende (4; 29) erstreckt;
Verbinden von wenigstens zwei Abschnitten des Kanals unter Verwendung eines Leiters (6; 31), der nicht-ferromagnetisches Material umfasst;
Verbinden einer Mehrzahl von Leitungen (8₁, 8₂, 8₃, 8₄, 8₅, 8₆; 33₁, 33₂, 33₃, 33₄), die entlang des Kanals voneinander beabstandet sind, mit dem Kanal, wobei das Bilden des Kanals, das Verbinden der wenigstens zwei Abschnitte des Kanals und das Verbinden der Mehrzahl von Leitungen mit dem Kanal umfassen:
Bereitstellen einer Schicht (23) aus Silizium;
Bemustern der Schicht derart, dass eine gemusterte Schicht (23') definiert wird, mit einem ersten Bereich, der dem Leiter (6; 31) entspricht, einem zweiten Bereich, der dem Kanal (2; 27) entspricht und weiteren Bereichen, die den Leitungen (8₁, 8₂, 8₃, 8₄, 8₅, 8₆; 33₁, 33₂, 33₃, 33₄) entsprechen; und Dotieren des ersten Bereichs.

47. Verfahren zum Betreiben einer Magnetowiderstandsvorrichtung mit einem Kanal (2; 27), umfassend nicht-ferromagnetisches Halbleitermaterial, das Silizium umfasst, wobei sich der Kanal von einem ersten Ende (3; 28) zu einem zweiten Ende (4; 29) erstreckt, einem Leiter (6; 31), der ein Metallsilizid oder Silizium mit einer höheren Leitfähigkeit als das Halbleitermaterial umfasst und wenigstens zwei Bereiche des Kanals verbindet, und einer Mehrzahl von Leitungen (8₁, 8₂, 8₃, 8₄, 8₅, 8₆; 33₁, 33₂, 33₃, 33₄), die entlang des Kanals voneinander beabstandet sind, wobei das Verfahren umfasst:
Fließenlassen von Strom zwischen nicht-benachbarten Leitungen (8₁, 8₅; 33₁, 33₃); und
Messen einer Spannung, die sich zwischen einer Leitung (8₃; 33₂), welche zwischen den nicht-benachbarten Leitungen liegt, und einer anderen Leitung (8₆; 33₄) entwickelt.

48. Verfahren nach Anspruch 47, wobei das Fließenlassen des Stroms zwischen nicht-benachbarten Leitungen (8₁, 8₅; 33₁, 33₃) ein Fließenlassen eines Stroms mit einer Stärke von wenigstens 1 µA umfasst.

49. Verfahren nach Anspruch 47 oder 48, ferner umfassend:
Anlegen eines magnetischen Felds (11) an die Magnetowiderstandsvorrichtung.

## Revendications

1. Dispositif (1 ; 26) à magnétorésistance ayant un canal (2 ; 27) comprenant un matériau semi-conducteur non ferromagnétique qui comprend du silicium, le canal s'étendant d'une première extrémité (3 ; 28) jusqu'à une deuxième extrémité (4 ; 29), un conducteur (6 ; 31) qui comprend un siliciure métallique ou du silicium ayant une conductivité plus élevée que le canal et reliant au moins deux tronçons du canal et une pluralité de fils conducteurs (8₁, 8₂, 8₃, 8₄, 8₅, 8₆ ; 33₁, 33₂, 33₃, 33₄) reliés au canal et espacés le long de ce dernier.

2. Dispositif selon la revendication 1, dans lequel le canal (2 ; 27) est un canal allongé.

3. Dispositif selon la revendication 1 ou 2, dans lequel le canal (2 ; 27) est incurvé.

4. Dispositif selon l'une quelconque des revendications précédentes, dans lequel le canal (2 ; 27) comprend du silicium-germanium.

5. Dispositif selon l'une quelconque des revendications précédentes, dans lequel le matériau semi-conducteur du canal est dopé avec une impureté ayant une concentration allant jusqu'à 1 x 10¹⁷ cm⁻³.

6. Dispositif selon l'une quelconque des revendications précédentes, dans lequel le matériau semi-conducteur du canal est dopé avec une impureté ayant une concentration allant jusqu'à 1 x 10¹⁸ cm⁻³.

7. Dispositif selon l'une quelconque des revendications précédentes, dans lequel le matériau semi-conducteur du canal est dopé avec une impureté ayant une concentration allant jusqu'à 1 x 10¹⁹ cm⁻³.

8. Dispositif selon l'une quelconque des revendications 1 à 4, dans lequel le matériau semi-conducteur est non dopé.

9. Dispositif selon l'une quelconque des revendications précédentes, dans lequel le canal (2 ; 27) est prévu dans une couche ayant une épaisseur inférieure à 50 nm.

10. Dispositif selon l'une quelconque des revendications précédentes, dans lequel le conducteur (6 ; 31) est agencé latéralement au canal (2 ; 27).

11. Dispositif selon l'une quelconque des revendications précédentes, dans lequel le conducteur (6 ; 31) s'étend le long du canal (2 ; 27).

12. Dispositif selon l'une quelconque des revendications précédentes, dans lequel le conducteur (6 ; 31) s'étend de la première extrémité (3 ; 28) jusqu'à la deuxième extrémité (4 ; 29) du canal (2 ; 27).

13. Dispositif selon l'une quelconque des revendications précédentes, comprenant une interface entre le conducteur (6 ; 31) et le canal (2 ; 27), l'interface étant divisée en au moins deux tronçons séparés.

14. Dispositif selon l'une quelconque des revendications précédentes, dans lequel le conducteur (6 ; 31) comprend un matériau semi-conducteur.

15. Dispositif selon l'une quelconque des revendications précédentes, dans lequel, si le conducteur comprend du silicium, le conducteur (6 ; 31) est dopé avec une impureté ayant une concentration d'au moins 1 x 10¹⁹ cm⁻³.

16. Dispositif selon l'une quelconque des revendications précédentes, dans lequel, si le conducteur comprend du siliciure métallique, le conducteur (6 ; 31) comprend du siliciure de titane.

17. Dispositif selon l'une quelconque des revendications précédentes, dans lequel le conducteur (6 ; 31) est prévu dans une couche ayant une épaisseur inférieure à 50 nm.

18. Dispositif selon l'une quelconque des revendications précédentes, dans lequel les fils conducteurs (8₁, 8₂, 8₃, 8₄, 8₅, 8₆ ; 33₁, 33₂, 33₃, 33₄) s'étendent latéralement par rapport au canal.

19. Dispositif selon l'une quelconque des revendications précédentes, dans lequel le conducteur (6 ; 31) est relié au canal (2, 27) le long d'un premier côté (5 ; 30) du canal.

20. Dispositif selon la revendication 19, dans lequel au moins certains des fils conducteurs (8₁, 8₂, 8₃, 8₄, 8₅, 8₆ ; 33₁, 33₂, 33₃, 33₄) sont reliés au canal (2 ; 37) sur un deuxième côté opposé (7 ; 32) du canal.

21. Dispositif selon la revendication 19 ou 20, dans lequel au moins certains des fils conducteurs (8₁, 8₂, 8₃, 8₄, 8₅, 8₆ ; 33₁, 33₂, 33₃, 33₄) sont reliés au canal (2 ; 37) sur une surface supérieure ou inférieure du canal.

22. Dispositif selon l'une quelconque des revendications précédentes, comprenant un premier, deuxième, troisième et quatrième fil conducteur (8₁, 8₂, 8₃, 8₄, 8₅, 8₆ ; 33₁, 33₂, 33₃, 33₄) agencé de manière ordonnée le long du canal.

23. Dispositif selon la revendication 22, dans lequel les premier et deuxième fils conducteurs (33₁, 33₂) sont espacés par une première séparation (s₁), les deuxième et troisième fils conducteurs (33₂, 33₃) sont espacés par une deuxième séparation (s₂) inférieure à la première séparation et les troisième et quatrième fils conducteurs (33₃, 33₄) sont espacés par une troisième séparation (s₃) inférieure à la première séparation.

24. Dispositif selon l'une quelconque des revendications précédentes, dans lequel les fils conducteurs (33₁, 33₂, 33₃, 33₄) comprennent un matériau semi-conducteur.

25. Dispositif selon la revendication 23, dans lequel les fils conducteurs (33₁, 33₂, 33₃, 33₄) comprennent le matériau semi-conducteur comprenant le canal (27).

26. Dispositif selon la revendication 24, dans lequel les fils conducteurs (33₁, 33₂, 33₃, 33₄) et le canal (27) sont unitaires.

27. Dispositif selon l'une quelconque des revendications 24 à 26, dans lequel les fils conducteurs (33₁, 33₂, 33₃, 33₄) comprennent du silicium.

28. Dispositif selon l'une quelconque des revendications 1 à 23, dans lequel les fils conducteurs (8₁, 8₂, 8₃, 8₄, 8₅, 8₆) comprennent du siliciure métallique.

29. Dispositif selon l'une quelconque des revendications 1 à 23, dans lequel les fils conducteurs (8₁, 8₂, 8₃, 8₄, 8₅, 8₆) comprennent du siliciure de titane.

30. Dispositif selon l'une quelconque des revendications 1 à 23, dans lequel les fils conducteurs comprennent un alliage ou métal non ferromagnétique.

31. Dispositif selon l'une quelconque des revendications précédentes, dans lequel les fils conducteurs ont chacun une épaisseur inférieure à 50 nm.

32. Dispositif selon l'une quelconque des revendications précédentes, dans lequel le canal (2 ; 27) a une largeur inférieure à 100 nm.

33. Dispositif selon l'une quelconque des revendications précédentes, dans lequel le canal (2 ; 27) a une longueur inférieure à 10 µm.

34. Dispositif selon l'une quelconque des revendications précédentes, dans lequel le conducteur (6 ; 31) a une largeur qui peut atteindre 500 nm.

35. Dispositif selon l'une quelconque des revendications précédentes, dans lequel le conducteur (6 ; 31) a une longueur inférieure à 10 µm.

36. Dispositif selon l'une quelconque des revendications précédentes, dans lequel les fils conducteurs (8₁, 8₂, 8₃, 8₄, 8₅, 8₆ ; 33₁, 33₂, 33₃, 33₄) ont chacun une largeur qui peut atteindre 200 nm, la largeur étant dans une direction qui correspond à la longueur pour le canal.

37. Dispositif selon l'une quelconque des revendications précédentes, comprenant en plus une base (9 ; 34) comprenant un matériau isolant, où le canal (2 ; 27) ; le conducteur (6 ; 31) et les fils conducteurs (8₁, 8₂, 8₃, 8₄, 8₅, 8₆ ; 33₁, 33₂, 33₃, 33₄) se trouvent sur la base.

38. Dispositif selon la revendication 37, comprenant en plus un substrat (10 ; 35), la base (9 ; 34) étant disposée sur le substrat.

39. Dispositif selon la revendication 38, comprenant en plus des régions de contact (37₁, 37₂, 37₃, 37₄) agencées dans le substrat (35) entre les fils conducteurs (33₁, 33₂, 33₃, 33₄) et les canaux (38₁, 38₂) entre les régions de contact sous les fils conducteurs de manière à procurer des transistors (39₁, 39₂) à effet de champ.

40. Dispositif selon l'une quelconque des revendications précédentes, comprenant en plus un couvercle comprenant un matériau isolant, dans lequel le couvercle se trouve sur le canal, la région et les fils conducteurs.

41. Dispositif selon l'une quelconque des revendications précédentes, comprenant en plus un ensemble de circuits (12) pour commander le dispositif.

42. Dispositif selon la revendication 41, dans lequel l'ensemble de circuits (12) est configuré pour faire circuler un courant électrique entre des fils conducteurs (8₁,8₅) non adjacents et pour mesurer une tension électrique développée entre un fil conducteur (8₃), entre les fils conducteurs non adjacents, et un autre fil conducteur (8₆).

43. Tête de lecture (1 ; 26) pour un lecteur (40) de disque dur comprenant le dispositif à magnétorésistance selon l'une quelconque des revendications précédentes.

44. Lecteur (40) de disque dur comportant au moins une tête de lecture (1 ; 26) selon la revendication 43.

45. Procédé de fabrication d'un dispositif à magnétorésistance, le procédé comprenant le fait de :
former un canal (2 ; 27) comprenant un matériau semi-conducteur non ferromagnétique qui comprend du silicium, le canal s'étendant d'une première extrémité (3 ; 28) jusqu'à une deuxième extrémité (4 ; 29) ;
relier au moins deux tronçons du canal en utilisant un conducteur (6 ; 31) comprenant un matériau non ferromagnétique ; et
relier au canal, une pluralité de fils conducteurs (8₁, 8₂, 8₃, 8₄, 8₅, 8₆ ; 33₁, 33₂, 33₃, 33₄) espacés le long du canal,
dans lequel le fait de former le canal, relier les au moins deux tronçons du canal et relier la pluralité de fils conducteurs au canal comprend le fait de :
prévoir une couche (23) de silicium ;
modeler ladite couche de manière à définir une couche (23') à motifs ayant une première région correspondant au conducteur (6 ; 31), une deuxième région correspondant au canal (2 ; 27) et des régions supplémentaires correspondant aux fils conducteurs (8₁, 8₂, 8₃, 8₄, 8₅, 8₆ ; 33₁, 33₂, 33₃, 33₄) ;
prévoir un métal (25₀) sur la première région ; et
exécuter un recuit de manière à former un siliciure métallique dans ladite première région.

46. Procédé de fabrication d'un dispositif à magnétorésistance, le procédé comprenant le fait de :
former un canal (2 ; 27) comprenant un matériau semi-conducteur non ferromagnétique qui comprend du silicium, le canal s'étendant d'une première extrémité (3 ; 28) jusqu'à une deuxième extrémité (4 ; 29) ;
relier au moins deux tronçons du canal en utilisant un conducteur (6 ; 31) comprenant un matériau non ferromagnétique ; et
relier au canal, une pluralité de fils conducteurs (8₁, 8₂, 8₃, 8₄, 8₅, 8₆ ; 33₁, 33₂, 33₃, 33₄) espacés le long du canal,
dans lequel le fait de former le canal, relier les au moins deux tronçons du canal et relier la pluralité de fils conducteurs au canal comprend le fait de :
prévoir une couche (23) de silicium ;
modeler ladite couche de manière à définir une couche (23') à motifs ayant une première région correspondant au conducteur (6 ; 31), une deuxième région correspondant au canal (2 ; 27) et des régions supplémentaires correspondant aux fils conducteurs (8₁, 8₂, 8₃, 8₄, 8₅, 8₆ ; 33₁, 33₂, 33₃, 33₄) ; et
doper ladite première région.

47. Procédé pour faire fonctionner un dispositif à magnétorésistance ayant un canal (2 ; 27) comprenant un matériau semi-conducteur non ferromagnétique qui comprend du silicium, le canal s'étendant d'une première extrémité (3 ; 28) jusqu'à une deuxième extrémité (4 ; 29), un conducteur (6 ; 31) qui comprend un siliciure métallique ou du silicium ayant une conductivité plus élevée que le matériau semi-conducteur et reliant au moins deux régions du canal et une pluralité de fils conducteurs (8₁, 8₂, 8₃, 8₄, 8₅, 8₆ ; 33₁, 33₂, 33₃, 33₄) espacés le long du canal, le procédé comprenant le fait de :
faire circuler un courant électrique entre des fils conducteurs (8₁, 8₅, 33₁, 33₃) non adjacents ; et
mesurer une tension électrique développée entre un fil conducteur (8₃, 33₂) qui se trouve entre les fils conducteurs non adjacents et un autre fil conducteur (8₆, 33₄).

48. Procédé selon la revendication 47, dans lequel la circulation du courant électrique entre les fils conducteurs (8₁, 8₅, 33₁, 33₃) non adjacents comprend le fait de faire circuler un courant électrique ayant une amplitude d'au moins 1µA.

49. Procédé selon la revendication 47 ou 48, comprenant en plus le fait :
d'appliquer un champ magnétique (11) au dispositif à magnétorésistance.
